# EUROPÄISCHE PATENTSCHRIFT

(11) **EP 1 196 393 B1**
(45) Veröffentlichungstag und Bekanntmachung des Hinweises auf die Patenterteilung: **02.04.2003**
(21) Anmeldenummer: 00942058.9
(22) Anmeldetag: 13.06.2000
(51) Int. Cl.: C07D 239/30

(54) **VERFAHREN ZUR HERSTELLUNG VON 4,6-DICHLORPYRIMIDIN**
METHOD FOR PRODUCING 4,6-DICHLOROPYRIMIDINE
PROCEDE DE PREPARATION DE 4,6-DICHLOROPYRIMIDINE

(30) Priorität: 26.06.1999 DE 19929353
(43) Veröffentlichungstag der Anmeldung: 17.04.2002
(73) Patentinhaber: Bayer Aktiengesellschaft, 51368 Leverkusen (DE)
(72) Erfinder: MAIS, Franz-Josef, D-40591 Düsseldorf (DE); CRAMM, Günther, D-51381 Leverkusen (DE); KLAUSENER, Alexander, D-50259 Pulheim (DE); STEFFAN, Guido, D-51519 Odenthal (DE)
(86) Internationale Anmeldenummer: EP0005416
(87) Internationale Veröffentlichungsnummer: WO01000591

(56) Entgegenhaltungen:
- EP-A- 0 095 637
- EP-A- 0 697 406
- EP-A- 0 745 593
- WO-A-95/29166
- WO-A-96/23776
- DE-A- 4 408 404
- DE-A- 19 531 299
- DE-A- 19 642 533
- GB-A- 2 325 224
- ANONYMOUS: "chlorination of pyrimidines" RESEARCH DISCLOSURE, Nr. 391, November 1996 (1996-11), Seiten 690-691, XP000680903 in der Anmeldung erwähnt
- TAKAO SAKAMOTO ET AL.: "Condensed Heteroaromatic Ring Systems VII. Synthesis of Thienopyridines, Thienopyrimidines, and Furopyridines from o-substituted N- Heteroarylacetylenes" CHEMICAL AND PHARMACEUTICAL BULLETIN, Bd. 34, Nr. 7, 1986, Seiten 2719-2724, XP002066903
- CARROLL TEMPLE, JR., ET AL.: "Preparation of 2,5-Diamino-4,6-dichloropyrimidine" JOURNAL OF ORGANIC CHEMISTRY, Bd. 40, Nr. 21, 1975, Seiten 3141-3142, XP000566489 columbus ohio
- JAMES GARNER ET AL.: "Regiocontrolled Amination of dichloropyrimidines in Liclo4-Et2O Solutions." HETEROCYCLIC COMMUNICATIONS, Bd. 5, Nr. 6, 1999, Seiten 503-508, XP000926047

## Beschreibung

Die vorliegende Erfindung betrifft ein Verfahren zur Herstellung von 4,6-Dichlorpyrimidin aus 4-Chlor-6-hydroxypyrimidin. 4,6-Dichlorpyrimidin ist ein wertvolles Zwischenprodukt zur Herstellung von Planzenschutzmitteln.

Es sind eine Reihe Verfahren zur Herstellung von 4,6-Dichlorpyrimidin bekannt, siehe beispielsweise WO96/23776, EP-A-697 406, EP-A-745 593, WO 95/29166, DE-A-19 53 129 und GB 2 325 224. Bei diesen Verfahren wird jedoch immer von 4,6-Dihydroxypyrimidin ausgegangen.

Es ist auch bekannt (siehe Res. Discl. n 391, 690-691 (1996)), daß man 4,6-Dichlorpyrimidin durch Umsetzung von 4-Chlor-6-methoxypyrimidin mit einem Chlorieragenz der Formel R₃PCl₂ umsetzen kann.

Die DE-A-44 08 404 beschreibt ein Verfahren zur Herstellung von Chlorpyrimidinen, unter anderem auch von 4,6-Dichlorpyrimidin. Als Ausgangsmaterial werden allgemein Hydroxypyrimidine genannt, nicht jedoch Chlorhydroxypyrimidine. Gemäß dieser Literaturstelle wird weiterhin mit POCl₃ unter Zusatz von Aminen oder Aminhydrochloriden chloriert.

Es ist noch kein Verfahren zur Herstellung von 4,6-Dichlorpyrimidin bekannt, bei dem man von 4-Chlor-6-hydroxypyrimidin ausgeht und in einfacher Weise das gewünschte Produkt erhält.

Es wurde nun ein Verfahren zur Herstellung von 4,6-Dichlorpyrimidin gefunden, das dadurch gekennzeichnet ist, daß man 4-Chlor-6-hydroxypyrimidin mit einem Säurechlorid umsetzt.

Als Säurechloride kommen organische und anorganische Säurechloride in Frage, beispielsweise PCl₃, POCl₃, PCl₅, R-PCl₂, R-PCl₄, R-POCl₂ und R₃PCl₂, wobei R für gegebenenfalls substituiertes C₆-C₁₀-Aryl oder gegebenenfalls substituiertes C₁-C₁₀-Alkyl steht, Säurechloride der Formel R'-CO-Cl mit R' = Chlor, C₁-C₁₀-Alkoxy, C₆-C₁₀-Aryloxy, -O-CCl₃, -CO-Cl, C₅-C₁₁-Heteroaryloxy mit 1 bis 3 Heteroatomen aus der Gruppe N, O und S, wobei die Alkoxy-, Aryloxy- und Hetaryloxyreste gegebenenfalls substituiert sein können sowie SOCl₂.

Die Säurechloride sind für sich alleine wirksam. Insbesondere sind keine Zusätze von Katalysatoren notwendig wie Amiden (etwa Diethylformamid), Aminen oder organischen Phosphorverbindungen (siehe EP-A-95 637).

Der Zusatz solcher grundsäztlich bekannter Katalysatoren ist jedoch möglich.

Es ist auch möglich Mischungen von Säurechloriden einzusetzen, dies ist jedoch nicht bevorzugt.

Es ist weiterhin möglich, das benötigte Säurechlorid in situ zu erzeugen. Beispielsweise kann man R₃PCl₂ aus R₃P und Chlor oder aus R₃P=O und einem Chloriermittel, z.B. PCl₃, Phosgen oder SOCl₂ erzeugen.

Es ist weiterhin möglich, nicht nur isoliertes 4-Chlor-6-hydroxypyrimidin einzusetzen, sondern auch ein Reaktionsgemisch, das 4-Chlor-6-hydroxypyrimidin enthält und beispielsweise aus der Spaltung von 4-Chlor-6-methoxypyridin stammt. Man kann das erfindungsgemäß einzusetzende Säurechlorid direkt in das Reaktionsgemisch aus der Spaltung von 4-Chlor-6-methoxypyrimidin eindosieren.

In das erfindungsgemäße Verfahren setzt man im allgemeinen mindestens 1 mol Säurechlorid pro Mol 4-Chlor-6-hydroxypyrimidin ein. Vorzugsweise beträgt diese Menge 1 bis 3 mol.

Als Lösungsmittel kommen grundsätzlich solche in Frage, die die durchzuführende Reaktion nicht negativ beeinflussen. Beispiele sind aliphatische Lösungsmittel wie Alkane, Cycloalkane und Halogenalkane, aromatische Lösungsmittel wie Benzol, Xylole, Toluol, Chlorbenzole, Benzotrifluorid, p-Chlorbenzotrifluorid und Anisol, wobei die aliphatischen und aromatischen Lösungsmittel gegebenenfalls weiter substituiert sein können, Nitrile wie Acetonitril und Benzonitril, N-haltige Lösungsmittel wie Dimethylformamid, Dimethylaceamid, Lactame und cyclische Harnstoffe und Ether und Polyether der verschiedensten Art. Auf Lösungsmittel kann man verzichten, wenn man flüssige Säurechloride, vorzugsweise im Überschuß einsetzt.

Das erfindungsgemäße Verfahren kann man z.B. bei Temperaturen im Bereich 0 bis 200°C, bevorzugt bei 20 bis 175°C, besonders bevorzugt bei 30 bis 150°C, durchführen. Der Druck ist nicht kritisch. Man kann beispielsweise bei 0,1 bis 50 bar, bevorzugt bei 0,5 bis 5 bar, arbeiten. Besonders bevorzugt arbeitet man bei Normaldruck.

Das erfindungsgemäße Verfahren kann in verschiedenen Ausführungsformen durchgeführt werden, beispielsweise diskontinuierlich, diskontinuierlich in Schüben oder kontinuierlich. Eine mögliche Verfahrensweise ist wie folgt: Man legt ein Säurechlorid gegebenenfalls mit einem Lösungsmittel vor und gibt 4-Chlor-6-hydroxypyrimidin zu. Danach kann man bei der gewünschten Temperatur rühren, bis die Umsetzung zum 4,6-Dichlorpyrimidin weitgehend oder vollständig abgelaufen ist. Man kann auch 4-Chlor-6-hydroxypyrimidin in Lösung oder als Suspension vorlegen und das Säurechlorid zudosieren. Auch andere Vorgehensweisen sind denkbar.

Die Aufarbeitung des nach der Reaktion vorliegenden Reaktionsgemisches kann beispielsweise durch Extraktion des hergestellten 4,6-Dichlorpyrimidins mit einem Lösungsmittel und anschließender Destillation des Extraktes erfolgen. Man kann auch das nach der Reaktion vorliegende Gemisch mit Wasser versetzen und dann 4,6-Dichlorpyrimidin abtrennen. Man kann auch das gesamte Reaktionsgemisch destillieren oder zunächst eine Rückchlorierung mit Cl₂/PCl₃ oder PCl₅ durchführen und anschließend destillieren. Auch andere Ausführungsformen und Aufarbeitungsmöglichkeiten sind denkbar.

Das erfindungsgemäße Verfahren zur Herstellung von 4,6-Dichlorpyrimidin ist wesentlich einfacher als die Verfahren des Standes der Technik. Es benötigt keine Katalysatoren oder Hilfsstoffe wie Amide, organische Phosphorverbindungen, Amine oder Amin-Hydrochloride. Es ist zudem bei Verwendung von flüssigen Säurechloriden ohne Lösungsmittel durchführbar, was die Aufarbeitung sehr vereinfacht.

### Beispiele

### Beispiel 1

In einem Rührgefäß wurden 100 Gew.-Teile Chlorbenzol, 13,1 Gew.-Teile 4-Chlor-6-hydroxypyrimidin und 36,6 Gew.-Teile Dichlortriphenylphosphoran vorgelegt. Danach erhitzte man unter Rühren auf 100°C und rührte 3 Stunden bei dieser Temperatur. Nach dem Abkühlen auf Raumtemperatur wurde mittels HPLC ein Gehalt von 9,95 Gew.-% 4,6-Dichlorpyrimidin im Reaktionsgemisch gefunden. Unter Berücksichtigung der Auswaage von 144,3 Gew.-Teilen an Reaktionsgemisch ergab sich somit eine Ausbeute von 96,7 % der Theorie. 4-Chlor-6-hydroxypyrimidin wurde im Reaktionsgemisch nur noch in Spuren gefunden.

### Beispiel 2

In einem Rührgefäß wurden 100 Gew.-Teile Thionylchlorid, 30 Gew.-Teile Triphenylphosphinoxid und 26,1 Gew.-Teile 4-Chlor-6-hydroxypyrimidin vorgelegt und unter Rühren auf Rückfluß erhitzt. Nach 6 Stunden wurde die Umsetzung abgebrochen und nach dem Abkühlen auf Raumtemperatur 130,1 Gew.-Teile Reaktionsgemisch erhalten, das mittels HPLC analysiert wurde. Es wurde ein Gehalt von 22,04 Gew.-% 4,6-Dichlorpyrimidin gefunden, entsprechend einer Ausbeute von 99,2 % der Theorie. 4-Chlor-6-hydroxypyrimidin war nach der Umsetzung nur noch in Spuren im Reaktionsgemisch enthalten.

### Beispiel 3

In einem Rührgefäß wurden 130 Gew.-Teile Phosphoroxychlorid und 26,1 Gew.-Teile 4-Chlor-6-hydroxypyrimidin vorgelegt und unter Rühren auf 100°C erhitzt. Nach 30 Minuten bei 100°C war die Umsetzung beendet. Die Auswaage an Reaktionsgemisch nach dem Abkühlen auf Raumtemperatur betrug 152,3 Gew.-Teile.

Seine Analyse mitels HPLC ergab einen Gehalt von 19,25 % 4,6-Dichlorpyrimidin, entsprechend einer Ausbeute von 98,4 % der Theorie.

Zur Aufarbeitung wurde das Reaktionsgemisch bei 50 bis 60°C fünfmal mit je 100 Gew.-Teilen Methylcyclohexan extrahiert. Die vereinigten Extrakte wurden im Vakuum eingedampft. Es blieb ein fester Rückstand von 30,8 Gew.-Teilen zurück. Dessen mittels HPLC ermittelter Gehalt an 4,6-Dichlorpyrimidin betrug 95,8 %, entsprechend einer Ausbeute von 99,0 % der Theorie.

### Beispiel 4

Es wurde verfahren wie in Beispiel 3 und nach dem Abkühlen ein Reaktionsgemisch in einer Auswaage von 152,8 Gew.-Teilen und mit einem mittels HPLC analysierten Gehalt an 4,6-Dichlorpyrimidin von 19,18 % erhalten, entsprechend einer Ausbeute von 98,3 % der Theorie.

Zur Aufarbeitung wurde das Reaktionsgemisch mit 33,0 Gew.-Teilen PCl₃ versetzt, auf 80°C erhitzt und unter Rühren im Verlauf von einer Stunde mit 14,2 Gew.-Teilen Chlor begast. Danach wurde zunächst bei Normaldruck, dann bei leichtem Vakuum (200 mbar) und einer Sumpftemperatur bis 65°C das Phosphoroxychlorid abdestilliert. Anschließend wurde bei 100 mbar destilliert. Man erhielt 4,6-Dichlorpyrimidin in einer Menge von 27,8 Gew.-Teilen mit einem Gehalt von 99,0 % (HPLC). Das entspricht einer Ausbeute von 92,4 % der Theorie.

### Beispiel 5

Es wurden 100 Gew.-Teile Dichlorphenylphosphinoxid und 20,08 Gew.-Teile 4-Chlor-6-hydroxypyrimidin vorgelegt und das Gemisch unter Rühren auf 100°C erhitzt. Nach 7 Stunden wurde unterbrochen und auf Raumtemperatur abgekühlt. Es wurden 116,0 Gew.-Teile Reaktionsgemisch erhalten, das gemäß HPLC-Anylyse einen Gehalt von 16,04 % 4,6-Dichlorpyrimidin und von 3,05 % 4-Chlor-6-hydroxypyrimidin aufwies. Das entspricht einer Ausbeute von 81,2 % 4,6-Dichlorpyrimidin und 17,6 % unumgesetztem Ausgangsprodukt.

### Beispiel 6

In einem Rührgefäß legte man 100 Gew.-Teile Chlorbenzol, 26,1 Gew.-Teile 4-Chlor-6-hydroxypyrimidin und 10 Gew.-Teile Dimethylformamid vor. Man erhitzte unter Rühren auf 100°C und leitete im Verlaufe von 4 Stunden 99 Gew.-Teile Phosgen gleichmäßig schnell ein. Danach leitete man bei 100°C 1 Stunde lang Stickstoff zum Ausblasen von Phosgenresten ein. Man kühlte auf Raumtemperatur und erhielt so 130,5 Gew.-Teile Reaktionsgemisch. Die HPLC-Analyse des Reaktionsgemisches ergab 19,8 % 4,6-Dichlorpyrimidin, entsprechend einer Ausbeute von 86,7 % der Theorie.

### Beispiel 7

In einem Rührgefäß wurden 110 Gew.-Teile Chlorbenzol, 26,1 Gew.-Teile 4-Chlor-6-hydroxypyrimidin und 45,8 Gew.-Teile Phosphorpentachlorid vorgelegt. Danach wurde unter Rühren auf 100°C erhitzt. Nach einer Stunde bei 100°C wurde auf Raumtemperatur abgekühlt und so 175,9 Gew.-Teile Reaktionsgemisch erhalten. Dessen HPLC-Analyse ergab einen Gehalt von 16,6 % 4,6-Dichlorpyrimidin, was eine Ausbeute von 98,0 % der Theorie entspricht.

### Beispiel 8

In einem Rührgefäß wurden 100 Gew.-Teile Acetonitril 14,5 Gew.-Teile 4-Chlor-6-methoxypyrimidin und 0,03 Gew.-Teile Wasser vorgelegt und unter Rühren bei 80°C im Verlaufe von 10 Stunden mit 37 Gew.-Teilen Chlorwasserstoff begast. Danach wurde eine HPLC-Probe genommen. Diese zeigte an, daß das 4-Chlor-6-methoxy pyrimidin nahezu vollständig umgesetzt und 4-Chlor-6-hydroxypyrimidin entstanden war.

Das so erhaltene Reaktionsgemisch wurde bei 80°C gerührt und im Verlaufe von 1 Stunde 30,7 Gew.-Teile Phosphoroxychlorid gleichmäßig schnell zugefügt. Nach 15 Minuten nachrühren wurde die Mischung im Vakuum eingeengt. Man erhielt einen braunen Rückstand, der dreimal mit je 5 Gew.-Teilen Methylcyclohexan extrahiert wurde. Das Einengen der vereinigten Methylcyclohexanextrakte ergab einen hellbeigen festen Rückstand an 4,6-Dichlorpyrimidin. Auswaage: 14,2 Gew.-Teile HPLC-Gehalt 98,9 %, entsprechend einer Ausbeute von 94,3 % der Theorie.

### Beispiel 9

Das Verfahren des Beispiels 8 wurde wiederholt. Nach der Zugabe von 30,7 Gew.-Teilen und 15-minütigem Nachrühren wurden 21 Gew.-Teile Phosphorpentachlorid portionsweise zugegeben. Man rührte noch 30 Minuten nach und destillierte den Ansatz komplett auf analoge Weise wie in Beispiel 4. Man erhielt 13,9 Gew.-Teile 4,6-Dichlorpyrimidin und einen HPLC-Gehalt von 99,1 %. Das entspricht einer Ausbeute von 92,4 % der Theorie.

## Patentansprüche

1. Verfahren zur Herstellung von 4,6-Dichlorpyrimidin, **dadurch gekennzeichnet, daß** man 4-Chlor-6-hydroxypyrimidin mit einem Säurechlorid umsetzt.

2. Verfahren nach Anspruch 1, **dadurch gekennzeichnet, daß** man als Säurechlorid PCl₃, POCl₃, PCl₅, R-PCl₂, R-PCl₄, R-POCl₂ und R₃PCl₂, wobei R für gegebenenfalls substituiertes C₆-C₁₀-Aryl oder gegebenenfalls substituiertes C₁-C₁₀-Alkyl steht, Säurechloride der Formel R'-CO-Cl mit R' = Chlor, C₁-C₁₀-Alkoxy, C₆-C₁₀-Aryloxy, -O-CCl₃, -CO-Cl, C₅-C₁₁-Heteroaryloxy mit 1 bis 3 Heteroatomen aus der Gruppe N, O und S, wobei die Alkoxy-, Aryloxy- und Hetaryloxyreste gegebenenfalls substituiert sein können sowie SOCl₂ einsetzt.

3. Verfahren nach Anprüchen 1 und 2, **dadurch gekennzeichnet, daß** man das benötigte Säurechlorid in situ erzeugt.

4. Verfahren nach Ansprüchen 1 bis 3, **dadurch gekennzeichnet, daß** man 4-Chlor-6-hydroxypyrimidin in isolierter Form oder in Form eines Reaktionsgemisches einsetzt, das 4-Chlor-6-hydroxypyrimidin enthält.

5. Verfahren nach Ansprüchen 1 bis 4, **dadurch gekennzeichnet, daß** man mindestens 1 mol Säurechlorid pro Mol 4-Chlor-6-hydroxypyrimidin einsetzt.

6. Verfahren nach Ansprüchen 1 bis 5, **dadurch gekennzeichnet, daß** man ein aliphatisches Lösungsmittel, ein aromatisches Lösungsmittel, ein Nitril, ein N-haltiges Lösungsmittel, einen Ether oder einen Polyether als Lösungsmittel einsetzt.

7. Verfahren nach Ansprüchen 1 bis 6, **dadurch gekennzeichnet, daß** man es bei Temperaturen im Bereich 0 bis 200°C durchführt.

8. Verfahren nach Ansprüchen 1 bis 7, **dadurch gekennzeichnet, daß** man es bei einem Druck im Bereich 0,1 bis 50 bar durchführt.

9. Verfahren nach Ansprüchen 1 bis 8, **dadurch gekennzeichnet, daß** man das Säurechlorid gegebenenfalls mit einem Lösungsmittel vorlegt und 4-Chlor-6-hydroxypyrimidin zufügt.

## Claims

1. Process for preparing 4,6-dichloropyrimidine, **characterized in that** 4-chloro-6-hydroxypyrimidine is reacted with an acid chloride.

2. Process according to Claim 1, **characterized in that** PCl₃, POCl₃, PCl₅, R-PCl₂, R-PCl₄, R-POCl₂ and R₃PCl₂, where R represents optionally substituted C₆-C₁₀-aryl or optionally substituted C₁-C₁₀-alkyl, acid chlorides of the formula R'-CO-Cl with R' = chlorine, C₁-C₁₀-alkoxy, C₆-C₁₀-aryloxy, -O-CCl₃, -CO-Cl, C₅-C₁₁-heteroaryloxy with 1 to 3 heteroatoms from the group N, O and S, where the alkoxy, aryloxy and hetaryloxy radicals may optionally be substituted, and SOCl₂ are employed as acid chloride.

3. Process according to Claims 1 and 2, **characterized in that** the required acid chloride is generated in situ.

4. Process according to Claims 1 to 3, **characterized in that** 4-chloro-6-hydroxypyrimidine is employed in isolated form or in the form of a reaction mixture containing 4-chloro-6-hydroxypyrimidine.

5. Process according to Claims 1 to 4, **characterized in that** at least 1 mol of acid chloride is employed per mole of 4-chloro-6-hydroxypyrimidine.

6. Process according to Claims 1 to 5, **characterized in that** an aliphatic solvent, an aromatic solvent, a nitrile, an N-containing solvent, an ether or a polyether is employed as solvent.

7. Process according to Claims 1 to 6, **characterized in that** it is carried out at temperatures in the range 0 to 200°C.

8. Process according to Claims 1 to 7, **characterized in that** it is carried out under a pressure in the range 0.1 to 50 bar.

9. Process according to Claims 1 to 8, **characterized in that** 4-chloro-6-hydroxypyrimidine is added to the acid chloride with, where appropriate, a solvent.

## Revendications

1. Procédé de préparation de 4,6-dichloropyrimidine, **caractérisé en ce que** l'on fait agir un chlorure d'acide sur de la 4-chloro-6-hydroxypyrimidine.

2. Procédé selon la revendication 1, **caractérisé en ce que** l'on utilise, comme chlorure d'acide, PCl₃, POCl₃, PCl₅, R-PCl₂, R-PCl₄, R-POCl₂ et R₃PCl₂, R représentant aryle en C₆-C₁₀ substitué le cas échéant ou alkyle en C₁-C₁₀ substitué le cas échéant, des chlorures d'acides de formule R'-CO-Cl, avec R' = chlore, alcoxy en C₁-C₁₀, aryloxy en C₆-C₁₀, -O-CCl₃, -CO-Cl, hétéroaryloxy en C₅-C₁₁ avec 1 à 3 hétéroatomes du groupe d'atomes N, O et S, les restes alcoxy, aryloxy et hétéroaryloxy pouvant être substitués le cas échéant, ainsi que SOCl₂.

3. Procédé selon les revendications 1 et 2, **caractérisé en ce que** l'on produit in situ le chlorure d'acide nécessaire.

4. Procédé selon les revendications 1 à 3, **caractérisé en ce que** l'on utilise la 4-chloro-6-hydroxypyrimidine sous forme isolée ou sous forme de mélange réactionnel qui contient la 4-chloro-6-hydroxypyrimidine.

5. Procédé selon les revendications 1 à 4, **caractérisé en ce que** l'on utilise au moins une mole de chlorure d'acide par mole de 4-chloro-6-hydroxypyrimidine.

6. Procédé selon les revendications 1 à 5, **caractérisé en ce que** l'on utilise, comme solvant, un solvant aliphatique, un solvant aromatique, un nitrile, un solvant azoté, un éther ou un polyéther.

7. Procédé selon les revendications 1 à 6, **caractérisé en ce qu'**on le réalise à des températures se situant dans une plage de 0 à 200°C.

8. Procédé selon les revendications 1 à 7, **caractérisé en ce qu'**on le réalise sous une pression se situant dans une plage de 0,1 à 50 bars.

9. Procédé selon les revendications 1 à 8, **caractérisé en ce que** l'on met d'abord en place le chlorure d'acide le cas échéant avec un solvant et qu'on ajouté la 4-chloro-6-hydroxypyrimidine.
